# EUROPEAN PATENT SPECIFICATION

(11) **EP 3 858 218 B1**
(45) Date of publication and mention of the grant of the patent: **27.09.2023**
(21) Application number: 21153679.2
(22) Date of filing: 27.01.2021
(51) Int. Cl.: A61B 1/00

(54) **ENDOSCOPE AND ENDOSCOPE ATTACHMENTS**
ENDOSKOP UND ENDOSKOPBEFESTIGUNGEN
ENDOSCOPE ET ATTACHES D'ENDOSCOPE

(30) Priority: 28.01.2020 US 202062966890 P
(43) Date of publication of application: 04.08.2021
(73) Proprietor: Gyrus ACMI, Inc. d/b/a Olympus Surgical Technologies America, Southborough MA 01772 (US)
(72) Inventor: EDWARDS, Kevin Cooper, Olive Branch, MS 38654 (US)
(74) Representative: Noack, Andreas

(56) References cited:
- EP-A1- 1 639 936
- US-A1- 2016 206 178
- US-A1- 2019 167 075
- US-B1- 6 352 503

## Description

### FIELD

These teachings relate to endoscopes or other medical devices with attachments. The teachings can provide irrigation, suction, cauterization, navigation, such as tracking of distal portion of a tool, or other function at a distal portion of the endoscope or other medical device.

### BACKGROUND

Traditionally, sinus surgeons hold an endoscope in a dedicated hand ("scope hand") and exchange instruments of various forms and functions with the other hand ("instrument hand") These instrument exchanges can be time consuming. The instrument exchange can be particularly important when managing bleeding such as when dissecting or resecting instruments are exchanged for irrigating, suctioning, or cauterizing instruments. Exemplary endoscopes are disclosed by US 2016/206178 A1, US 2019/167075 A1, EP 1639936 A1 and US 6352503 B1.

### SUMMARY

These teachings can help overcome one or more of the problems with prior devices discussed in the Background, such as by providing an endoscope with an attachment device that can be rotatable about a perimeter of an endoscope shaft.

For example, an endoscope can include an elongated shaft. An optical illumination carrier can provide light to a distal end of the shaft. A light-receiving optical carrier can receive light incident on the distal end of the shaft and provide such received light to a proximal portion of the endoscope. An attachment device can be mechanically coupled to the shaft such that the attachment device can be rotatable about a perimeter of the shaft.

The attachment device, when mechanically coupled to the shaft, can be non-concentric and laterally coupled and offset from the shaft. The attachment device can include a tubular structure. The tubular structure can include an outer surface facing an outer surface of the shaft. The attachment device can extend along the shaft. The attachment device can be positionable such that a portion of the attachment device extends beyond a distal end of the shaft.

An opening in the attachment device can include a distal opening that is more distal than the distal end of the shaft. The endoscope can include a collar such as can provide the mechanical coupling between the attachment device and the shaft. The collar can be attached to the attachment device and at least partially situated around a perimeter of the shaft. The attachment device can include at least one of an irrigation tube, a suction tube, a navigation device, or an electrode. A second attachment device can be mechanically coupled to the shaft, such that the second attachment device can be axially translatable along and rotatable about a perimeter of the shaft.

These teachings can provide an attachment device for an endoscope (or another medical device). The attachment device can include an elongated structure. The attachment device can include a mechanical coupling device such as integrally formed with or mechanically coupled to the elongated structure. The mechanical coupling device can be configured to allow an endoscope to translate therethrough and mechanically couple the attachment device to the endoscope. The attachment device can be laterally coupled to and offset from a shaft of the endoscope. The elongate structure can include a hollow tubular structure.

The elongated structure, when mechanically coupled to the endoscope, can include an outer surface facing an outer surface of a shaft of the endoscope. The elongated structure is coupled to a pump or electrical power device. The attachment device, when mechanically coupled to the endoscope, can be rotatable about a perimeter of a shaft of the endoscope. The attachment device, when mechanically coupled to the endoscope, can be axially translatable along the shaft of the endoscope.

These teachings can, without forming part of the claimed invention, provide a method of using a medical device. The method can include mechanically coupling an attachment device to an elongated shaft of the medical device such that the attachment device is laterally coupled and offset from the shaft. The method can include operating the attachment device while a distal portion of the attachment device is in the opening and while the medical device illuminates a structure accessible through the opening. The method can include axially translating the attachment device along the shaft while the attachment device is partially situated in the opening.

The method can include rotating the attachment device about a perimeter of the shaft before operating the attachment device. Operating the attachment device can include irrigating the structure or suctioning the structure. Operating the attachment device can include at least one of cutting the structure using electricity or cauterizing the structure. Operating the attachment device can include determining a location of a distal end of the attachment device within the opening.

The invention is defined by the appended claims. Any embodiment, example or teaching that is disclosed herein but is contradictory to the claims, is provided for exemplary purpose only and does not form part of the claimed invention.

### BRIEF DESCRIPTION OF THE DRAWINGS

FIG. 1 illustrates, by way of example, a diagram of an embodiment of an endoscope system.
FIGS. 2, 3, and 4 illustrate, by way of example, respective diagrams of embodiments of hand tools.
FIGS. 5, 6, and 7 illustrate, by way of example, diagrams of embodiments of respective attachment devices that can be used in place of the hand tools of FIGS. 2, 3, and 4, respectively.
FIG. 8 illustrates, by way of example, a diagram of an embodiment of an endoscope system that includes an attachment device mechanically coupled to an endoscope.
FIG. 9 illustrates, by way of example, a diagram of an embodiment of the endoscope system of FIG. 8 after the attachment device is translated along a shaft of the endoscope.
FIG. 10 illustrates, by way of example, a diagram of an embodiment of the endoscope system of FIG. 8 after the attachment device is rotated about a perimeter of the shaft.
FIG. 11 illustrates, by way of example, a diagram of an embodiment of the endoscope system of FIG. 8 after the attachment device is rotated about a perimeter of the shaft and axially translated along the shaft.
FIG. 12 illustrates, by way of example, a diagram of an embodiment of an endoscope system that includes multiple attachment devices mechanically coupled to the endoscope or a single attachment device with multiple functions.
FIG. 13 illustrates an example of an embodiment of an endoscope system that is keyed.
FIG. 14 illustrates an example of an embodiment of an endoscope system that includes the attachment device attached about a lens cleaning sheath.
FIG. 15 illustrates an example of an embodiment of a method for operating an endoscope system, such as the endoscope system of the teaching.

### DETAILED DESCRIPTION

These teachings can provide a medical device, such as an endoscope, such as in a system with a rotatable attachment device. An endoscope can provide optical illumination, such as internal to a patient. An endoscope can be a slender and tubular instrument. An endoscope can be used to look inside a patient. The procedure of viewing inside a patient body is called an endoscopy. The endoscope can be used to examine internal organs like a throat, sinus cavity, or esophagus. An endoscope can be specialized, such as to view a target organ. Such specialized endoscopes can be named after their target organ. For example, a sinuscope is specialized to provide a view of a sinus cavity, an otoscope is specialized to provide a view of an inner ear, a laryngoscope is specialized to provide a view of a larynx, a cystoscope is specialized to provide a view of the bladder, a nephroscope is specialized to view the kidney, a bronchoscope is specialized to view the bronchus, an arthroscope is specialized to view a joint, a colonoscope is specialized to view a colon, and a laparoscope is specialized to view an abdomen or pelvis. The endoscope can be used to visually examine and diagnose. The endoscope can be used to assist in surgery or other medical procedures. This document explains, among other things, how to modify an endoscope to provide further surgical assistance such as by providing an endoscope with an attachment device that can be rotatable about a perimeter of an endoscope shaft.

For example, an endoscope can include an elongated shaft. The endoscope can further include an optical illumination carrier element such as to provide light to a distal end of the shaft. The endoscope can further include an optical carrier element such as to provide light incident on the distal end of the shaft. The endoscope can further include an attachment device that can be mechanically coupled to the shaft such that the attachment device is rotatable about a perimeter of the shaft. The attachment device, when mechanically coupled to the shaft, can be non-concentric and laterally coupled to and offset from the shaft.

The attachment device can include a tubular or non-tubular structure. The tubular structure can include an outer surface facing an outer surface of the shaft. The attachment device can extend along the shaft. The attachment device can be positionable beyond a distal end of the shaft. An opening in the attachment device can include a distal opening that is more distal than the distal end of the shaft. A non-tubular structure can include one or more elements, such as a lever, spring, switch, push rod, or the like, that can translate an electrode, navigation probe, or manipulate tissue.

The endoscope can further include a collar that provides the mechanical coupling between the attachment device and the shaft. The collar can be attached to the attachment device and at least partially situated around a perimeter of the shaft. The attachment device can include at least one of an irrigation tube, a suction tube, a navigation device, or an electrode. The endoscope can further include a second attachment device mechanically coupled to the shaft such that the second attachment device is axially translatable along and rotatable about a perimeter of the shaft.

A method of using an endoscope can include mechanically coupling an attachment device to an elongated shaft of the endoscope such that the attachment device is laterally coupled and offset from the shaft. The method can include operating the attachment device while a distal portion of the attachment device is in the opening and while the endoscope illuminates a structure accessible through the opening. The method can further include axially translating the attachment device along the shaft while the attachment device is partially situated in the opening. The method can further include rotating the attachment device about a perimeter of the shaft before operating the attachment device.

Operating the attachment device can include irrigating the structure or suctioning the structure. Operating the attachment device can include at least one of cutting the structure using electricity or cauterizing the structure. Operating the attachment device can include determining a location of a distal end of the attachment device within the opening.

Reference will now be made to the FIGS. to describe embodiments and further details.

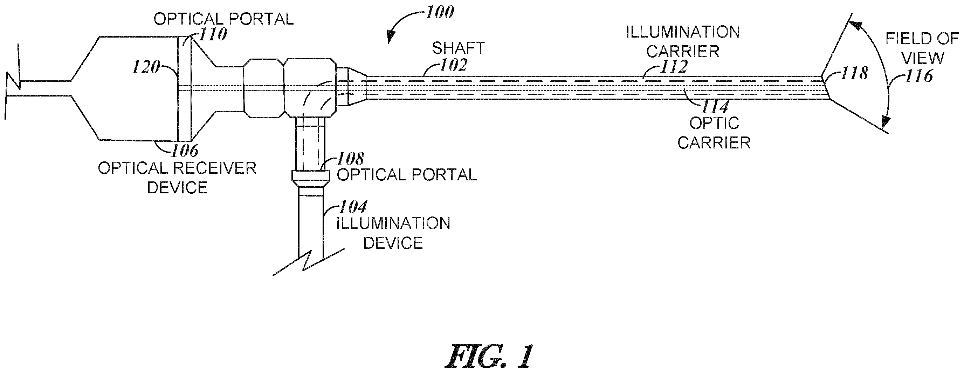

FIG. 1 illustrates an example of an endoscope system. The endoscope system can include an endoscope 100, an optical illumination device 104, and an optional optical receiver device 106. The optical illumination device 104 can convert an electrical signal to an optical signal. The optical illumination device 104 can provide the optical signal to an optic carrier 114 of the endoscope 100. The optical illumination device 104 can include or be coupled to an optical emitter, such as a laser, light emitting diode (LED), or the like.

The optical receiver device 106 can convert an optical signal carried by the optic carrier 114 into an electrical signal. The optical receiver device 106 can include a charge coupled device (CCD), a fiber optic receiver, or the like. An optical portal 110 can include a lens such as can help magnify the signal. A user can then view what is in a field of view 116 of the endoscope 100 through the lens. When included, the optical receiver device 106 can provide electrical signals that can be converted to pixel values and provided for viewing such as on a display device.

The endoscope 100 as illustrated can include an optical portal 108 such as to receive optical illumination from the illumination device 104. The optical portal 108 can include an illumination carrier 112 extending thereto. The illumination carrier 112 can communicate illumination light from the illumination device 104 to a distal end 118 of the endoscope 100. The illumination light can illuminate an object in the field of view 116. The illumination carrier 112 can include one or more optical fibers, mirrors, lenses, reflective surfaces, or the like, such as along a path that guides the light to the distal end 118.

The endoscope 100 can include another optical portal 110. The optic carrier 114 can carry incident light from within the field of view 116 to a proximal end 120 of the endoscope 100. The optic carrier 114 can include one or more optic fibers, mirrors, lenses, reflective surfaces, or the like.

A shaft 102 of the endoscope 100 can house the illumination carrier 112 and the optic carrier 114. The optic carrier 114 can be located within the illumination carrier 112 in the shaft 102. The shaft 102 can be rigid, flexible, or a combination thereof. For example, the shaft 102 can be rigid, entirely flexible, or can include some rigid and some flexible portions.

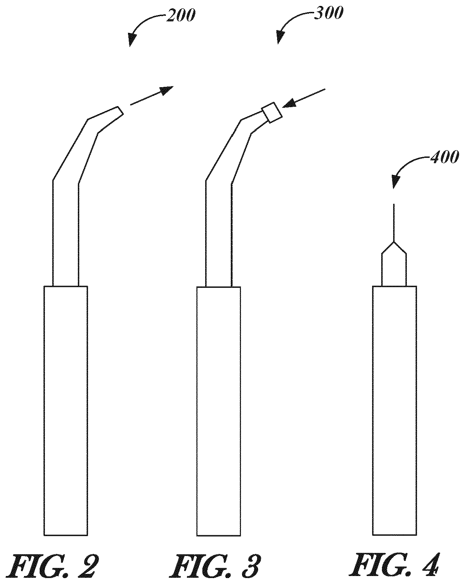

FIGS. 2, 3, and 4 illustrate examples of hand tools. FIG. 2 illustrates an example of an irrigation device 200. FIG. 3 illustrates an example of a suction device 300. FIG. 4 illustrates an example of a cautery or ablation device 400. The irrigation device 200 can be used to provide liquid from a reservoir to a target structure or target location. The suction device 300 can be used to remove material from around the target structure or target location. The cautery or ablation device 400 can be used to modify the target structure, such as by cutting or coagulating target tissue.

In practice, the endoscope 100 can be held by a user, such as in a first hand of the user. The user can insert a distal end 118 of the endoscope 100 into an opening in a patient and to a target location within the patient and beyond the opening. The illumination device 104 can provide illumination light that is guided by the illumination carrier 112 to the target location. Light incident on the optic carrier 114 is guided to the optical receive device 106 or a lens. Such incident received light can provide the user with a view of a structure at the target location.

The user, with the view provided by the optic carrier 114, can translate a hand tool (see FIGS. 2, 3, and 4 for examples of hand tools) with their second hand into the opening. This typically occurs while the user is still holding the endoscope in their first hand. The user can use the view of the structure to inform whether the hand tool is operating in a proper location or where to operate the hand tool, for example.

The user can then operate the hand tool in the opening, such as to alter a structure via the opening. Examples of the operations performed by the hand tools can include suction (removing loose material), irrigation (adding liquid), cauterizing (burning, such as to stop bleeding or removing tissue), ablating (destroying tissue), navigating (identifying location), or the like. To change hand tools, the user can retract the hand tool out of the opening, set the hand tool down, retrieve a second hand tool, and insert the second hand tool into the opening. The user then operates the second hand tool in the opening, such as aided by the view provided by the optical receiver device 106. This process can be repeated until the user has altered the structure at the target location to their liking.

This process can be difficult to the user and can require fine motor skills and steady hands, such as to perform small, fine movements around the structure and through the opening in a person. The process can be improved using one or more attachment devices that can be connected to the endoscope 100. The attachment device can provide the functionality of the hand tool, without requiring the user to continue to hold the hand tool steady in a second hand while holding the endoscope steady in their first hand.

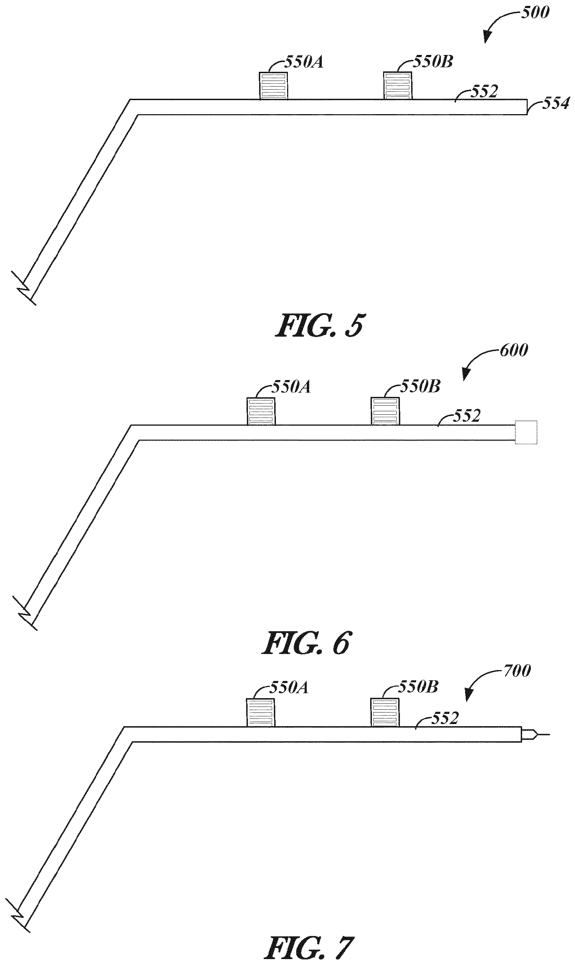

FIGS. 5, 6, and 7 illustrate examples of respective attachment devices 500, 600, and 700. The attachment devices 500, 600, 700 can perform similar functions as the attachment devices 200, 300, 400, respectively. However, the attachments devices 500, 600, 700 do not require a second hand of the user to move the device near the structure, operate, or remove from the opening.

The attachment devices 500, 600, 700 can include a mechanical coupling device 550A, 550B. The mechanical coupling device 550A, 550B can be slid over the shaft 102. The mechanical coupling device can then be tightened to form a friction or compression coupling between the mechanical coupling device 550A, 550B and the shaft 102. The mechanical coupling device 550A, 550B can include a collar, such as can be tightened or loosened by turning a screw, a compliant grommet or O-ring sized and shaped to fit over the shaft, or the like.

The mechanical coupling device 550A, 550B can be mechanically attached to a tube 552. The tube 552 can have a circular, elliptical, square, triangular, other polygonal, irregular, or other shaped cross-section. The tube 552 can carry liquid to a distal end 554 thereof in the embodiment of FIG. 5. The tube 552 can carry material away from the structure in the embodiment of FIG. 6. The tube 552 can house electrical interconnects and be connected to an electrical power supply to cauterize or ablate the structure in the embodiment of FIG. 7. The tube can house a vaporizer device that can be translated to extend beyond the distal end 118 of the endoscope 102 and perform cautery.

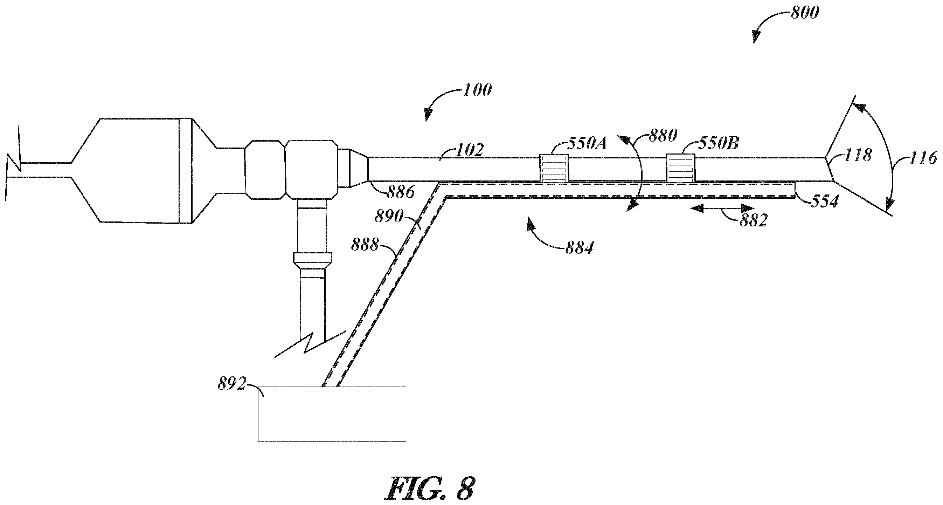

FIG. 8 illustrates an example of an endoscope system 800. The endoscope system 800 is similar to the endoscope system of FIG. 1 but includes an attachment device 884 mechanically coupled to the endoscope 100 and another device 892 coupled to the attachment device 884. The attachment device 884 illustrated is an irrigation device, but can be a cautery device, a navigation device, a suction device, or the like.

The attachment device 884 can include the mechanical coupling devices 550A, 550B. The mechanical coupling devices 550A, 550B can extend outwardly from an outer surface 888 of the attachment device 884. The mechanical coupling devices 550A, 550B can be laterally coupled to the attachment device 884. The mechanical coupling devices 550A, 550B can be slid over the distal end 118 of the shaft 102. The mechanical coupling devices 550A, 550B can be tightened to form a compression or friction coupling to the shaft 102. The mechanical coupling devices 550A, 550B can be loosened or the coupling to the shaft 102 can inherently allow the attachment device 884 to be translated along the shaft 102 as indicated by arrow 882. The mechanical coupling devices 550A, 550B can be loosened or the coupling to the shaft 102 can inherently allow the attachment device 884 to be rotated about a perimeter of the shaft 102 as indicated by arrow 880. While two mechanical coupling devices 550A, 550B are shown in FIG. 8, the more or fewer mechanical coupling devices 550A, 550B can be used to mechanically couple the attachment device 884 to the shaft 102.

The mechanical coupling devices 550A, 550B can include a threaded collar and screw. The screw when turned a first direction (e.g., clockwise or anti-clockwise) can tighten the mechanical coupling device 550A, 550B around the shaft 102. The screw, when turned a second direction, opposite the first direction, can loosen the mechanical coupling device 550A, 550B. The mechanical coupling devices 550A, 550B can include an O-ring that stretches to fit around the shaft 102. When released, the O-ring can compress causing a compression or friction fit with the shaft 102. The mechanical coupling devices 550A, 550B can include a clasp, pin, or the like. The clasp can be opened allowing the endoscope to be inserted into the clasp. The clasp can then be closed, at least partially enclosing the shaft 102. The mechanical coupling devices 550A, 550B can be integrally formed with, or releasably mechanically coupled to the attachment device 884. The mechanical coupling devices 550A, 550A can at least partially surround the shaft 102, such as to partially surround the shaft 102 or completely surround the shaft 102.

Where the shaft 102 is flexible, the attachment device 884 can add rigidity. The attachment device 884 can thus limit the flexibility of the shaft 102.

After the attachment device 884 is mechanically coupled to the shaft 102, an outer surface of the shaft 886 faces an outer surface 888 of the attachment device 884. After the attachment device 884 is mechanically coupled to the shaft 102, the attachment device 884 is laterally coupled to and offset from the shaft 102. After mechanical coupling, some prior endoscope cleaning devices include an outer surface of the shaft 886 facing an inner surface of the cleaning device.

The attachment device 884 can include a tubular structure 890. The tubular structure 890 can be hollow or solid. A hollow tubular structure 890 can allow liquid, material suctioned from around the structure, an electrical interconnect, or other material to travel therethrough. The tubular structure 890 can include a transverse cross-section (generally perpendicular to longitudinal) that includes a round, rectangular, triangular, other polygonal shape, or an irregular shape.

The device 892 can include a pump, liquid reservoir, a holding tank, an electrical power generator, a display, a combination thereof, or the like. The attachment device 884 can be electrically or mechanically coupled to the device 892. The attachment device 884 can receive liquid from the device 892 and provide the liquid out a distal end of the attachment device 884, such as to irrigate the structure. A pump of the device 892 can cause a vacuum to remove material from on or around the structure. The device 892 can be coupled to an electrical interconnect (e.g., a wire, trace, or other electrically conductive mechanism). The device 892 can provide electrical energy to an electrode on a distal end 554 of the attachment device 884, such as for cautery, ablation, or the like. The device 892 can help a user determine whether a distal end 118 of the shaft 102 is in a proper location. The device 892 can provide an augmented or unaugmented view of the field of view 116. The navigation can include a determination of proximity to a sensor situated at a specified location about the structure. The navigation can include a correlation of pixel data of the field of view 116 with another image.

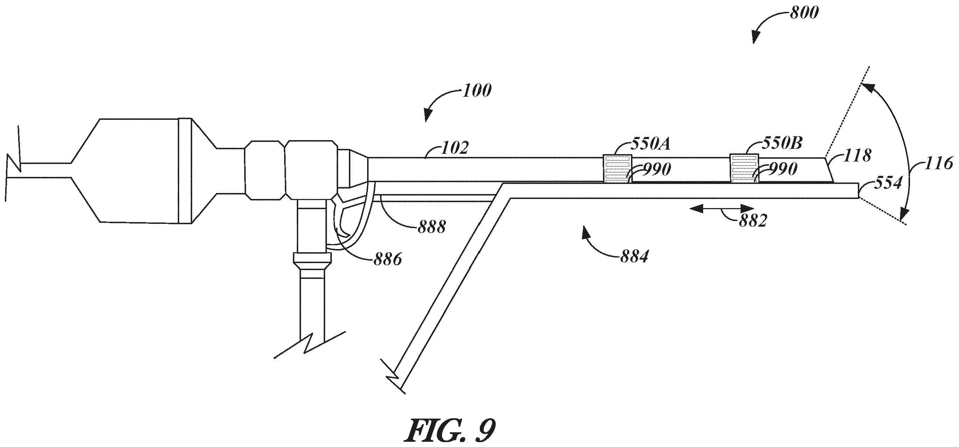

FIG. 9 illustrates an example of the endoscope system 800 after the attachment device 884 is translated along the shaft 102. The user can cause the translation by pressing a translation switch, such as a button, lever, trigger, switch, or the like. The endoscope 100 can include a switch 886 that moves a push rod 888 to translate the attachment device 884 along the shaft 102. The translation can be assisted using a spring, lever, or other pushing device. The spring, or another device, can assist translation of the attachment device 884 back to its position along the shaft before translation (as shown in FIG. 8). The arrow 882 indicates the directions of translation. Translating the attachment device 884 along the shaft 102 moves the distal end 554 of the attachment device 884 relative to the distal end 118 of the shaft 102. The distance between the distal ends 554, 118 can be increased or decreased by translating the attachment device 884 along the shaft 102. The attachment device 884 can be translated along the shaft such that the distal end 554 is within the field of view 116. The attachment device 884 can be translated such that the distal end 554 is more distal than the distal end 118. In other words, the attachment device 884 can be translated such that the attachment device 884 extends beyond the distal end 118 of the shaft 102.

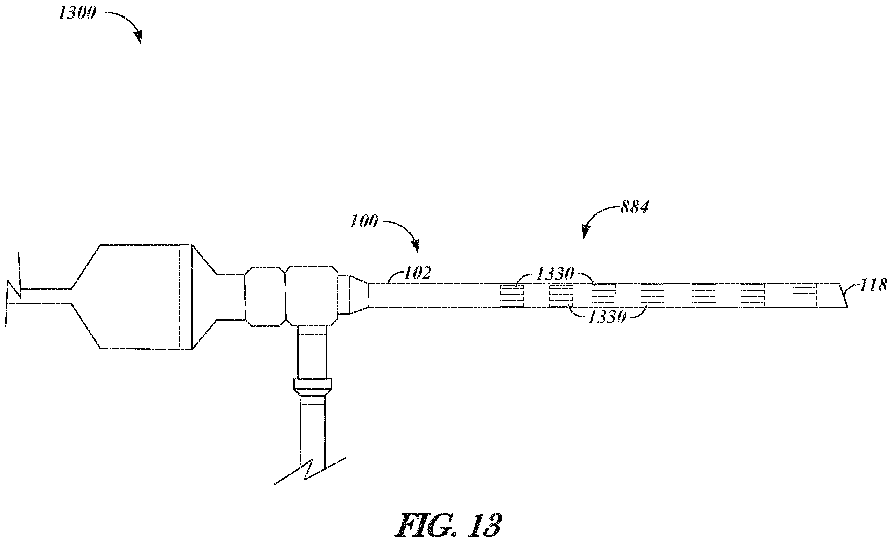

The collar 550A, 550B can include a male or female connection feature 990. The male or female connection feature 990 can be configured to mate with a female or male connection feature 1330 on the shaft 102 (see FIG. 13). The male or female connection feature 990 can include an indent, bump, slot, socket, ball, clip, or the like. The user can lock the attachment device 884 at a predetermined location along the shaft by mating the male or female connection feature 990 with the female or male connection feature 1330.

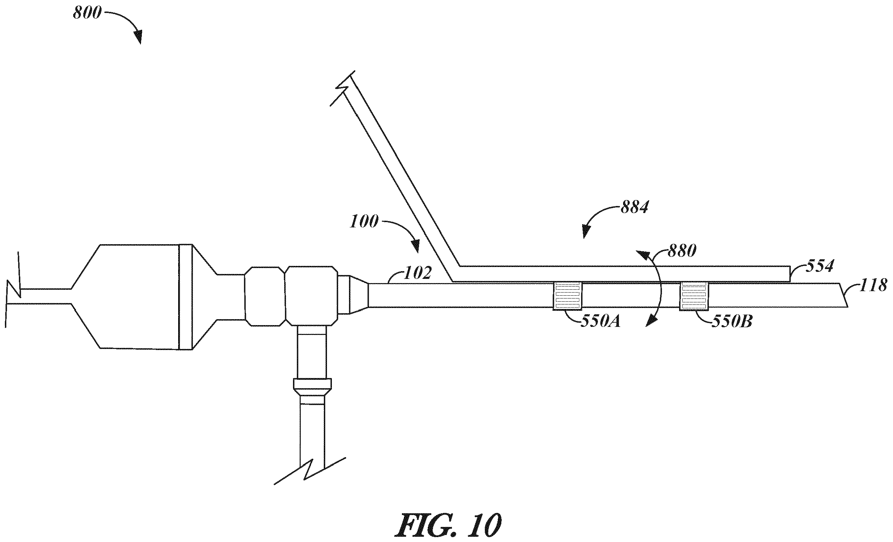

FIG. 10 illustrates an example of the endoscope system 800 after the attachment device 884 is rotated about a perimeter of the shaft 102. The arrow 880 indicates the directions of rotation. Rotating the attachment device 884 about a perimeter of the shaft 102 moves an orientation of attachment device 884 relative to the shaft 102. The attachment device 884 can be situated above, below, on the side of the shaft 102, or any location therebetween.

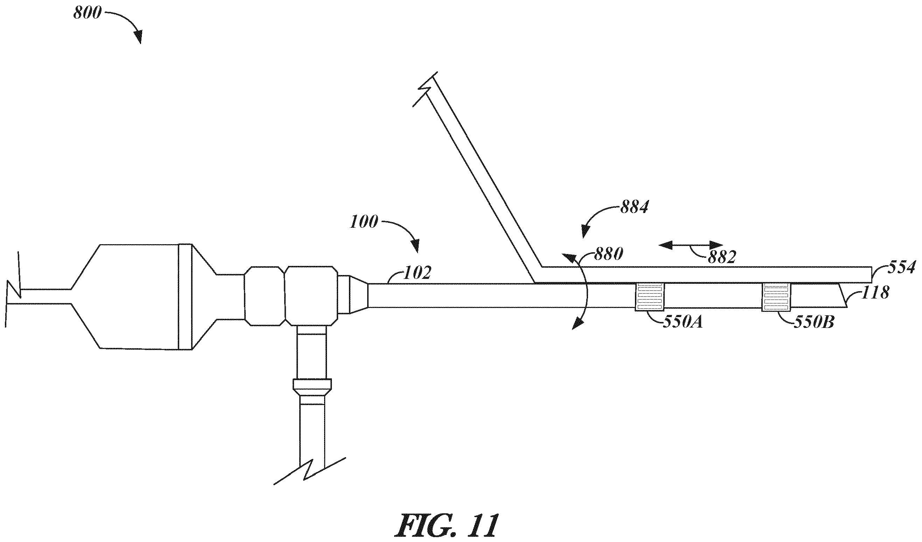

FIG. 11 illustrates an example of the endoscope system 800 after the attachment device 884 is rotated about a perimeter of the shaft 102 and translated along the shaft 102. The arrow 880 indicates the directions of rotation and the arrow 882 indicates the directions of translation.

FIGS. 8-11 illustrate an example of a single attachment device 884 mechanically coupled to the shaft 102. In some use cases, it can be beneficial to have multiple attachment devices mechanically coupled to the shaft 102. Multiple attachment devices can allow the user to perform multiple operations on the structure. For example, a suction attachment device and an irrigation attachment device can be coupled to the shaft 102. This can allow the user to spray liquid on or around the structure using the irrigation attachment device and remove excess liquid or other material from around the structure using the suction attachment device. In an example, a cautery attachment device and a navigation attachment device, suction attachment device, or irrigation attachment device can be coupled to the shaft 102. This can allow the user to cauterize or ablate the structure and ensure they are cauterizing or ablating the proper location or cleaning on or around the structure.

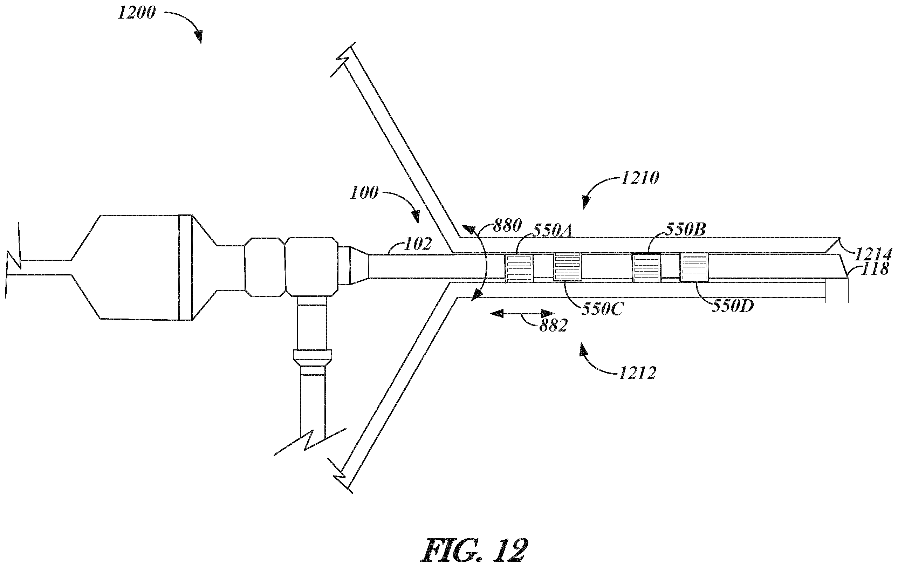

FIG. 12 illustrates an example of an embodiment of an endoscope system 1200 that includes multiple attachment devices 1210, 1212 mechanically coupled to the endoscope 100. The attachment devices 1210, 1212 can be similar to one or more of the attachment devices 500, 600, 700, 884. The attachment device 1210, 1212 can be rotatable about the perimeter of the shaft 102 or translatable along the shaft 102. The attachment devices 1210, 1212 can include a suction device and an irrigation device. The irrigation device 1210 can be situated to provide liquid to the distal end 118 of the shaft 102. The liquid can clean a lens or other optical component on or near the distal end 118 of the shaft 102. The suction device 1212 can operate to remove the liquid from the irrigation attachment device 1210 and the debris or other material loosened by the liquid. The attachment devices 1210, 1212 can be translated towards the structure and then operated to irrigate and remove material. The translation can be performed using a single hand. The endoscope system 1200 can be removed from the opening or otherwise translated away from the structure using a single hand.

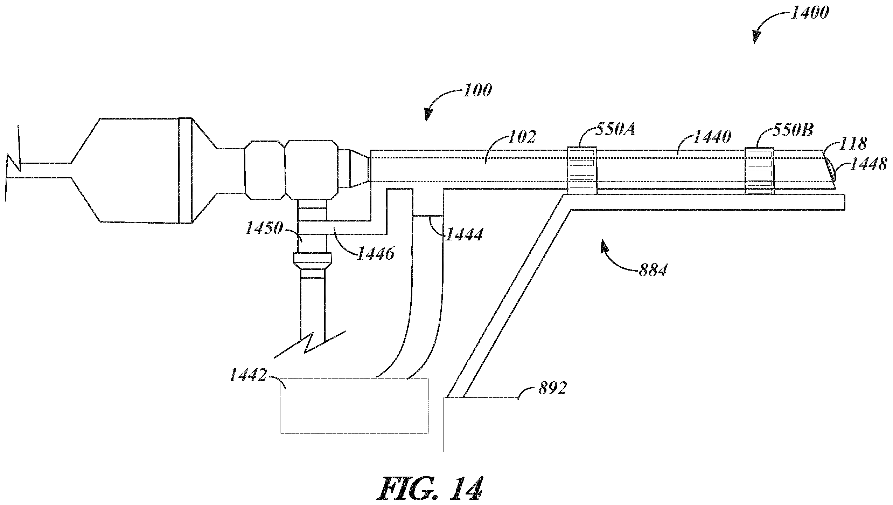

FIG. 13 illustrates an example of an embodiment of an endoscope system 1300 that is keyed, such as to maintain the rotational position, such as at one of several pre-specified locking points. The key of the endoscope system 1300 can include a female or male connection feature 1330 that mates with the male or female connection feature 990 (see FIG. 9) of the collar 550A, 550B. The key can be configured to lock the attachment device 884 in position and resist attachment device slipping. FIG. 14 illustrates an example of an embodiment of an endoscope system 1400 that includes the attachment device 884 mechanically coupled about a perimeter of a lens cleaning sheath 1440. The attachment device 884 can, alternatively, be integrally formed with the lens cleaning sheath 1440.

The lens cleaning sheath 1440 can include a fluid portal 1444. The fluid portal 1444 can receive fluid from a fluid reservoir 1442. The lens cleaning sheath 1440 can direct the fluid to the distal end 118 of the endoscope 110. The fluid can remove debris from the distal end 118 of the endoscope 100, such as to clean the optic carrier 114, illumination carrier 112 (see FIG. 1), or a lens 1448 on the distal end 118 of the endoscope 100. The lens 1448 can alter a path of light incident thereon. From the perspective of light from the illumination carrier 112 the lens 1448 can be concave and can spread the light. From the perspective of light outside the endoscope 100, the lens is convex and can focus the light on the optic carrier 114.

The sheath 1440 can include a stabilizer 1446 that helps retain the sheath 1440 in a proper orientation and position relative to the endoscope 100. The proper orientation and position is one that allows the fluid to be incident on the lens 1448. The stabilizer 1446 can help prevent the sheath 1440 from rotating by a mechanically coupling to an arm 1450 of the endoscope 100 coupled to the illumination device 104. The sheath 1440 can include an O-ring, grommet, or the like, that provides a friction or compression fit to the perimeter of the shaft 102. The O-ring, grommet, or other mechanical coupling can help prevent the sheath 1440 from translating along the shaft 102.

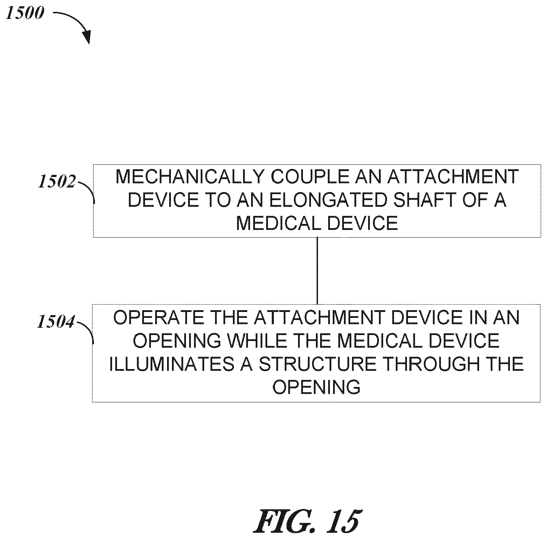

FIG. 15 illustrates an example of an embodiment of a method 1500 for operating an endoscope system, such as the endoscope system 800, 1200, 1300, 1400, or the like. The method 1500 as illustrated includes mechanically coupling an attachment device 884 to an elongated shaft 102 of the medical device (such that the attachment device is laterally coupled and offset from the shaft), at operation 1502; and operating the attachment device 884 while a distal portion of the attachment device 884 is in the opening and while the medical device illuminates a structure accessible through the opening, at operation 1504.

The method 1500 can further include axially translating the attachment device 884 along the shaft 102 while the attachment device 884 is partially situated in the opening. The method 1500 can further include rotating the attachment device 884 about a perimeter of the shaft 102 before operating the attachment device 884. Operating the attachment device 884 can include irrigating the structure or suctioning the structure. Operating the attachment device 884 can include at least one of cutting the structure using electricity or cauterizing the structure. Operating the attachment device 884 can include determining a location of a distal end of the attachment device 884 within the opening.

The method steps disclosed herein can be performed in any order except as specified otherwise. Moreover, one or more of the method steps can be combined with other steps; can be omitted or eliminated; can be repeated; and/or can separated into individual or additional steps.

The explanations and illustrations presented herein are intended to acquaint others skilled in the art with the invention, its principles, and its practical application. The above description is intended to be illustrative and not restrictive. Those skilled in the art may adapt and apply the invention in its numerous forms, as may be best suited to the requirements of a particular use.

Accordingly, the embodiments of the present invention as set forth are not intended as being exhaustive or limiting of the teachings. Further, components of the specific embodiments can be combined with components of other embodiments of the teachings. The scope of the teachings should, therefore, be determined not with reference to this description, but should instead be determined with reference to the appended claims, along with the full scope of equivalents to which such claims are entitled. The omission in the following claims of any aspect of subject matter that is disclosed herein is not a disclaimer of such subject matter, nor should it be regarded that the inventors did not consider such subject matter to be part of the disclosed inventive subject matter.

Plural elements or steps can be provided by a single integrated element or step. Alternatively, a single element or step might be divided into separate plural elements or steps. The disclosure of "a" or "one" to describe an element or step is not intended to foreclose additional elements or steps. While the terms first, second, third, etc., may be used herein to describe various elements, components, regions, layers and/or sections, these elements, components, regions, layers and/or sections should not be limited by these terms. These terms may be used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

## Claims

1. An endoscope comprising:
an elongated shaft (102);
an optical illumination carrier to (112) provide light to a distal end (118) of the shaft (102);
an optical carrier (114) to provide light incident on the distal end (118) of the shaft (102) to a proximal portion (120) of the endoscope (100); and
a first attachment device (1210) mechanically coupled to the shaft (102) by means of first mechanical coupling devices (550A, 550B), allowing the first attachment device (1210) to be rotatable about a perimeter of the shaft (102), and
a second attachment device (1212) mechanically coupled to the shaft (102) by means of second mechanical coupling devices (550C, 550D), allowing the second attachment device (1212) to be axially translatable along and rotatable around a perimeter of the shaft (102).

2. The endoscope of claim 1, wherein the attachment devices (1210, 1212), when mechanically coupled to the shaft (102), are non-concentric and laterally coupled and offset from the shaft (102).

3. The endoscope of claim 1 or 2, wherein the attachment devices (1210, 1212) include a tubular structure (890).

4. The endoscope of claim 3, wherein the tubular structure (890) includes an outer surface (888) facing an outer surface of the shaft (102).

5. The endoscope of any of claims 1 to 3, wherein the attachment devices (1210, 1212) extend along the shaft (102) and are positionable such that portions of the attachment devices (1210, 1212) extend beyond a distal end (118) of the shaft (102).

6. The endoscope of claim 5, wherein an opening in the attachment devices (1210, 1212) includes a distal opening that is more distal than the distal end (118) of the shaft (102).

7. The endoscope of any of claims 1 to 6, further comprising collars that provide the mechanical coupling between the attachment devices (1210, 1212) and the shaft (102), the collars attached to the attachment devices (1210, 1212) and at least partially situated around a perimeter of the shaft (102).

8. The endoscope of any of claims 1 to 7, wherein the attachment devices (1210, 1212) include at least one of an irrigation tube, a suction tube, a navigation device, or an electrode.

9. The endoscope of any of claims 1 to 8, further comprising a male or female connection feature (1330) situated on the shaft and configured to mate with a corresponding female or male connection feature (990) of the attachment devices (1210, 1212) and maintain position of the attachment device (1210, 1212) at a pre-specified location.

## Patentansprüche

1. Ein Endoskop, umfassend:
einen langgestreckten Schaft (102);
einen optischen Beleuchtungsträger (112), um einem distalen Ende (118) des Schafts (102) Licht zuzuführen;
einen optischen Träger (114), um Licht, das auf das distale Ende (118) des Schafts (102) fällt, an einen proximalen Abschnitt (120) des Endoskops (100) zu führen; und
eine erste Befestigungsvorrichtung (1210), die mechanisch mit dem Schaft (102) mittels erster mechanischer Kupplungsvorrichtungen (550A, 550B) gekoppelt ist, wodurch die erste Befestigungsvorrichtung (1210) um einen Umfang des Schafts (102) drehbar ist, und
eine zweite Befestigungsvorrichtung (1212), die mechanisch mit dem Schaft (102) mittels zweiter mechanischer Kupplungsvorrichtungen (550C, 550D) gekoppelt ist, wodurch die zweite Befestigungsvorrichtung (1212) entlang eines Umfangs des Schafts (102) axial verschiebbar und um diesen drehbar ist.

2. Das Endoskop nach Anspruch 1, wobei die Befestigungsvorrichtungen (1210, 1212), wenn sie mechanisch mit dem Schaft (102) gekoppelt sind, nicht-konzentrisch und seitlich gekoppelt und von dem Schaft (102) versetzt sind.

3. Das Endoskop nach Anspruch 1 oder 2, wobei die Befestigungsvorrichtungen (1210, 1212) eine rohrförmige Struktur (890) umfassen.

4. Das Endoskop nach Anspruch 3, wobei die röhrenförmige Struktur (890) eine Außenfläche (888) aufweist, die einer Außenfläche des Schafts (102) gegenüberliegt.

5. Das Endoskop nach einem der Ansprüche 1 bis 3, wobei sich die Befestigungsvorrichtungen (1210, 1212) entlang des Schafts (102) erstrecken und so positionierbar sind, dass Teile der Befestigungsvorrichtungen (1210, 1212) über ein distales Ende (118) des Schafts (102) hinausragen.

6. Das Endoskop nach Anspruch 5, wobei eine Öffnung in den Befestigungsvorrichtungen (1210, 1212) eine distale Öffnung umfasst, die weiter distal liegt als das distale Ende (118) des Schafts (102).

7. Das Endoskop nach einem der Ansprüche 1 bis 6, weiterhin umfassend Kragen, die die mechanische Kopplung zwischen den Befestigungsvorrichtungen (1210, 1212) und dem Schaft (102) bereitstellen, wobei die Kragen an den Befestigungsvorrichtungen (1210, 1212) angebracht sind und sich zumindest teilweise um einen Umfang des Schafts (102) herum befinden.

8. Das Endoskop nach einem der Ansprüche 1 bis 7, wobei die Befestigungsvorrichtungen (1210, 1212) mindestens eines der folgenden Elemente umfassen: einen Spülschlauch, einen Saugschlauch, eine Navigationsvorrichtung oder eine Elektrode.

9. Das Endoskop nach einem der Ansprüche 1 bis 8, weiterhin umfassend ein männliches oder weibliches Verbindungsmerkmal (1330), das sich auf dem Schaft befindet und dazu eingerichtet ist, mit einem entsprechenden weiblichen oder männlichen Verbindungsmerkmal (990) der Befestigungsvorrichtungen (1210, 1212) zusammenzupassen und die Position der Befestigungsvorrichtung (1210, 1212) an einer vordefinierten Stelle zu halten.

## Revendications

1. Un endoscope comprenant :
un tube allongé (102) ;
un porteur d'éclairage optique pour (112) fournir de la lumière à une extrémité distale (118) du tube (102) ;
un porteur optique (114) pour fournir de la lumière incidente sur l'extrémité distale (118) du tube (102) à une partie proximale (120) de l'endoscope (100) ; et
un premier dispositif de fixation (1210) couplé mécaniquement au tube (102) au moyen de premiers dispositifs de couplage mécanique (550A, 550B), permettant au premier dispositif de fixation (1210) d'être rotatif autour d'un périmètre du tube (102), et
un deuxième dispositif de fixation (1212) couplé mécaniquement au tube (102) au moyen de deuxièmes dispositifs de couplage mécanique (550C, 550D), permettant au deuxième dispositif de fixation (1212) d'être axialement translatable le long et rotatif autour d'un périmètre du tube (102).

2. L'endoscope de la revendication 1, dans lequel les dispositifs de fixation (1210, 1212), lorsqu'ils sont couplés mécaniquement au tube (102), sont non-concentriques et couplés latéralement et décalés par rapport au tube (102).

3. L'endoscope de la revendication 1 ou 2, dans lequel les dispositifs de fixation (1210, 1212) comprennent une structure tubulaire (890).

4. L'endoscope de la revendication 3, dans lequel la structure tubulaire (890) comprend une surface extérieure (888) faisant face à une surface extérieure du tube (102).

5. L'endoscope de l'une des revendications 1 à 3, dans lequel les dispositifs de fixation (1210, 1212) s'étendent le long du tube (102) et sont positionnables de sorte que des parties des dispositifs de fixation (1210, 1212) s'étendent au-delà d'une extrémité distale (118) du tube (102).

6. L'endoscope de la revendication 5, dans lequel une ouverture des dispositifs de fixation (1210, 1212) comprend une ouverture distale qui est plus distale que l'extrémité distale (118) du tube (102).

7. L'endoscope de l'une des revendications 1 à 6, comprenant en outre des colliers qui assurent le couplage mécanique entre les dispositifs de fixation (1210, 1212) et le tube (102), les colliers fixés aux dispositifs de fixation (1210, 1212) et au moins partiellement situés autour d'un périmètre du tube (102).

8. L'endoscope de l'une des revendications 1 à 7, dans lequel les dispositifs de fixation (1210, 1212) comprennent au moins un parmi un tube d'irrigation, un tube d'aspiration, un dispositif de navigation ou une électrode.

9. L'endoscope de l'une quelconque des revendications 1 à 8, comprenant en outre une caractéristique de connexion mâle ou femelle (1330) située sur le tube et configurée pour s'accoupler avec une caractéristique de connexion femelle ou mâle correspondante (990) des dispositifs de fixation (1210, 1212) et maintenir la position du dispositif de fixation (1210, 1212) à un emplacement spécifié à l'avance.
